# FASCICULE DE BREVET EUROPEEN

(11) **EP 4 077 511 B1**
(45) Date de publication et mention de la délivrance du brevet: **02.09.2026**
(21) Numéro de dépôt: 20817361.7
(22) Date de dépôt: 07.12.2020
(51) Int. Cl.: C08J 11/24, C08L 67/02

(54) **PROCÉDÉ D'OBTENTION D'UN EFFLUENT DIESTER PURIFIE PAR DÉPOLYMÉRISATION D'UN POLYESTER COMPRENANT DU POLYÉTHYLÈNE TÉRÉPHTALATE OPAQUE**
VERFAHREN ZUR HERSTELLUNG EINES GEREINIGTEN DIESTERABSTROMS DURCH DEPOLYMERISATION EINES POLYESTERS MIT OPAKEM POLYETHYLENTEREPHTHALAT
METHOD FOR OBTAINING A PURIFIED DIESTER EFFLUENT BY DEPOLYMERISING A POLYESTER COMPRISING OPAQUE POLYETHYLENE TEREPHTHALATE

(30) Priorité: 19.12.2019 FR 1914992
(43) Date de publication de la demande: 26.10.2022
(73) Titulaire: IFP Energies nouvelles, 92852 Rueil-Malmaison (FR)
(72) Inventeur: CHICHE, David, 92852 RUEIL-MALMAISON (FR); LEINEKUGEL LE COCQ, Damien, 92852 RUEIL-MALMAISON CEDEX (FR); BOUNIE, Christine, 92852 RUEIL-MALMAISON CEDEX (FR)
(74) Mandataire: IFP Energies nouvelles
(86) Numéro de dépôt international: PCT/EP2020/084851
(87) Numéro de publication internationale: WO 2021/122096

(56) Documents cités:
- EP-A2- 0 080 434
- WO-A1-2018/007356

## Description

### Domaine technique

L'invention concerne un procédé de dépolymérisation par glycolyse d'un polyester, en particulier le polyester téréphtalate, comprenant du polyéthylène téréphtalate (PET) coloré et/ou opaque et/ou multicouches, en vue de son recyclage dans une unité de polymérisation. Plus particulièrement, l'invention concerne un procédé de dépolymérisation par glycolyse d'une charge polyester comprenant au moins du PET coloré et/ou opaque, avec une étape de purification finale de l'effluent diester obtenu optimisée.

### Technique antérieure

Le recyclage chimique de polyester, en particulier du polyéthylène téréphtalate (PET), a fait l'objet de nombreux travaux visant à décomposer le polyester récupéré sous forme de déchets en monomères qui pourront de nouveau être utilisés comme charge d'un procédé de polymérisation.

De nombreux polyesters sont issus de circuits de collecte et de tri de matières. En particulier, le polyester, en particulier le PET, peut provenir de la collecte de bouteilles, barquettes, films, résines et/ou fibres composées de polyester (comme par exemple des fibres textiles, des fibres de pneus). Le polyester issu de filières de collecte et de tri est appelé polyester à recycler.

Le PET à recycler peut être classé en quatre grandes catégories :
- le PET clair, constitué majoritairement de PET transparent incolore (en général au moins 60% poids) et de PET transparent coloré azuré, qui ne contient pas de pigments et peut être engagé dans des procédés de recyclage mécanique,
- le PET foncé, ou coloré (vert, rouge,..), qui peut contenir généralement jusqu'à 0,1% poids de colorants ou pigments mais reste transparent, ou translucide ;
- le PET opaque, qui contient une quantité significative de pigments à des teneurs variant typiquement entre 0,25 et 5,0% poids pour opacifier le polymère. Le PET opaque est utilisé de manière grandissante par exemple pour la fabrication de contenants alimentaires, comme les bouteilles de lait, dans la composition de flacons cosmétiques, phytosanitaires ou de colorants ;
- le PET multicouches, qui comporte des couches de polymères autres que le PET ou une couche de PET recyclé entre des couches de PET vierge (c'est-à-dire PET n'ayant pas subi de recyclage), ou un film d'aluminium par exemple. Le PET multicouches est utilisé après thermoformage pour faire des emballages tels que des barquettes.

Les filières de collecte, qui alimentent les filières de recyclage, sont structurées différemment en fonction des pays. Elles évoluent de manière à maximiser la quantité de plastique valorisé dans les déchets en fonction de la nature et de la quantité des flux et des technologies de tri. La filière de recyclage de ces flux est en général constituée d'une première étape de conditionnement sous forme de paillettes au cours de laquelle des balles d'emballage brut sont lavées, purifiées et triées, broyées puis de nouveau purifiées et triées pour produire un flux de paillettes contenant en général moins de 1% massique d'impuretés « macroscopiques » (verre, métaux, autres plastiques, bois, papier carton, éléments minéraux), préférentiellement moins de 0,2% d'impuretés « macroscopiques » et encore plus préférentiellement moins de 0,05%.

Les paillettes de PET clair peuvent ensuite subir une étape d'extrusion-filtration permettant de produire des extrudés qui sont ensuite réutilisables en mélange avec du PET vierge pour faire de nouveaux produits (bouteilles, fibres, films). Une étape de polymérisation sous vide à l'état solide (connu sous l'acronyme SSP) est nécessaire pour les usages alimentaires. Ce type de recyclage est appelé recyclage mécanique.

Les paillettes de PET foncé (ou coloré) sont également recyclables mécaniquement. Cependant, la coloration des extrudés formés à partir des flux colorés limite les usages : le PET foncé est le plus souvent utilisé pour produire des fibres ou des lanières d'emballage. Les débouchés sont donc plus limités par rapport à ceux du PET clair.

La présence de PET opaque contenant des pigments à des teneurs importantes, dans le PET à recycler, pose des problèmes aux recycleurs car le PET opaque altère les propriétés mécaniques du PET recyclé. Le PET opaque est actuellement collecté avec le PET coloré et se retrouve dans le flux de PET coloré. Compte tenu du développement des usages du PET opaque, les teneurs en PET opaque dans le flux de PET coloré à recycler sont actuellement comprises entre 5-20% poids et ont tendance à augmenter encore. D'ici quelques années, il sera possible d'atteindre des teneurs en PET opaque dans le flux de PET coloré supérieures à 20-30% poids. Or il a été montré qu'au-delà de 10-15% de PET opaque dans les flux de PET coloré, les propriétés mécaniques du PET recyclé sont altérées (cf. *« Impact du développement du PET opaque blanc sur le recyclage des emballages en PET »,* note préliminaire du COTREP du 5/12/13) et empêchent le recyclage sous forme de fibres, principal débouché de la filière pour le PET coloré.

Les colorants sont des substances naturelles ou synthétiques, solubles notamment dans la matière polyester et utilisés pour colorer la matière dans laquelle ils sont introduits. Les colorants généralement utilisés sont de différentes natures et contiennent souvent des hétéroatomes de type O et N, et des insaturations conjuguées, comme par exemple des fonctions quinone, methine, azo, ou des molécules comme la pyrazolone et la quinophtalone. Les pigments sont des substances finement divisées, insolubles en particulier dans la matière polyester, utilisés pour colorer et/ou opacifier la matière dans laquelle ils sont introduits. Les principaux pigments utilisés pour colorer et/ou opacifier les polyesters, en particulier le PET, sont des oxydes métalliques comme TiO₂, CoAl₂O₄, Fe₂O₃, des silicates, des polysulfides, et du noir de carbone. Les pigments sont des particules de taille comprise généralement entre 0,1 et 10 µm, et en majorité entre 0,4 et 0,8 µm. L'élimination totale de ces pigments par filtration, nécessaire pour envisager un recyclage du PET opaque, est techniquement difficile car ils sont extrêmement colmatants.

Le recyclage des PET colorés et opaques est donc extrêmement délicat.

La demande de brevet US 2006/0074136 décrit un procédé de dépolymérisation par glycolyse de PET coloré, en particulier issu de la récupération de bouteilles de PET colorées vertes. La charge traitée par ce procédé se présente sous forme de paillettes de PET et est mise en contact d'éthylène glycol dans un réacteur à une température entre 180 et 280°C pendant plusieurs heures. Le produit de glycolyse obtenu à l'issue l'étape de dépolymérisation est purifié sur charbon actif à une température supérieure à 170°C puis par extraction des colorants résiduels, en particulier les colorants jaune, par un solvant qui peut être un alcool tel que le méthanol, ou un glycol tel que l'éthylène glycol. Le BHET quant à lui cristallise dans le solvant d'extraction et est alors séparé par filtration.

Dans la demande de brevet US 2015/0105532, le PET post-consommation comprenant un mélange de différents PET colorés, comme du PET clair, du PET bleu, du PET vert et/ou du PET ambré, sous forme de paillettes, est dépolymérisé par glycolyse en présence d'éthylène glycol et d'un catalyseur amine et d'alcool, dans un réacteur à 150-250°C, en mode batch. Le monomère diester alors obtenu est purifié par filtration, échange d'ions et/ou passage sur charbon actif en particulier à une température de 80-90°C, avant d'être cristallisé et récupéré par filtration.

Le brevet US 6,642,350 décrit quant à lui la purification d'une solution de BHET brute comprenant au minimum une succession de mises en contact de ladite solution avec un charbon actif, une résine échangeuse d'anions et une résine échangeuse de cations, en particulier à une température égale à 60°C, 65°C ou 80°C. Ce brevet montre en effet qu'une simple mise en contact uniquement avec du charbon actif ne suffit pas notamment à décolorer entièrement la solution puisqu'une couleur résiduelle, en particulier jaune, est observée lorsque ladite solution de BHET brute est mise en contact uniquement avec du charbon actif.

D'autres techniques antérieures sont connues à partir de WO2018/007356.

Enfin, la demande de brevet FR 3053691 décrit un procédé de dépolymérisation d'une charge polyester comprenant du PET opaque et en particulier de 0,1 à 10% poids de pigments, par glycolyse en présence d'éthylène glycol. Un effluent de bis-(2-hydroxyethyl) téréphtalate (BHET) purifié est obtenu après des étapes particulières de séparation et de purification par adsorption. Cependant, l'effluent BHET obtenu par le procédé décrit dans la demande FR 3053691 peut présenter des imperfections : l'effluent BHET obtenu se colore notamment rapidement malgré le passage sur une colonne d'adsorbant.

La présente invention cherche à perfectionner ces procédés de dépolymérisation par glycolyse d'une charge polyester comprenant du PET coloré et/ou opaque et en particulier celui de la demande FR 3053691, notamment afin d'améliorer la purification, plus particulièrement la décoloration, de l'effluent diester obtenu après séparation des impuretés lourdes et solides, comme les oligomères et les pigments. L'objectif de l'invention est en effet d'obtenir un flux de diester, en particulier un flux de BHET, par dépolymérisation d'une charge polyester comprenant du PET coloré et/ou opaque, avec une pureté élevée et étant en particulier incolore ou pratiquement incolore.

### Résumé de l'invention

L'invention a donc pour objet un procédé de dépolymérisation d'une charge polyester comprenant du polyéthylène téréphtalate (PET) opaque, ledit procédé comprenant :
a) une étape de conditionnement comprenant une section de conditionnement alimentée au moins par ladite charge polyester, pour produire un flux conditionné ;
b) une étape de dépolymérisation par glycolyse alimentée au moins par le flux conditionné et par un appoint de diol de sorte que la quantité molaire de diol est ajustée entre 1 à 20 moles de diol par mole de diester de ladite charge polyester, opérée à une température comprise entre 180 et 400°C, et un temps de séjour compris entre 0,1 et 10 h ;
c) une étape de séparation du diol alimentée au moins par l'effluent de l'étape b), opérée à une température comprise entre 100 et 250°C, à une pression inférieure à celle de l'étape b) et produisant un effluent diol et un effluent riche en monomères liquide, ladite étape de séparation du diol étant mise en œuvre dans 1 à 5 sections de séparation gaz-liquide successives produisant chacune un effluent gaz et un effluent liquide, l'effluent liquide de la section antérieure alimentant la section ultérieure, l'effluent liquide issu de la dernière section de séparation gaz-liquide constituant l'effluent riche en monomères liquide, l'ensemble des effluents gaz étant récupéré pour constituer l'effluent diol ;
d) une étape de séparation de l'effluent riche en monomères liquides issu de l'étape c) en un effluent impuretés lourdes et un effluent monomères pré-purifié, opérée à une température inférieure à 250°C et une pression inférieure à 0,001 MPa avec un temps de séjour liquide inférieur à 10 min, et
e) une étape de décoloration de l'effluent monomères pré-purifié, comprenant au moins une section de mélange de l'effluent monomères pré-purifié issu de l'étape d) avec un solvant de sorte que l'effluent monomères pré-purifié représente entre 20 et 90% poids du poids total du mélange, et une section d'adsorption opérée à une température comprise entre 100 et 200°C, et à une pression comprise entre 0,1 et 1,0 MPa, en présence d'au moins un adsorbant choisi parmi les charbons actifs, les alumines et les argiles, pour produire un effluent monomères purifié.

Un avantage de la présente invention réside en l'obtention, à partir d'une charge polyester comprenant au moins du PET, en particulier du PET coloré et/ou opaque, d'un effluent diester, en particulier un flux BHET, purifié, étant en particulier incolore, voire quasi incolore.

Un intérêt de l'invention est donc de pouvoir traiter tout type de déchets polyester, qui comprennent de plus en plus de pigments et colorants, comme les PET colorés, opaques, voire multi-couches. Le procédé selon l'invention, apte à traiter du PET opaque, permet de retirer les pigments et colorants et de revenir au monomère diester par réaction chimique. Ce monomère peut ensuite être repolymérisé en un polymère qui ne présente aucune différence avec un polyester vierge, en particulier un PET vierge, autorisant ainsi tous les usages du PET vierge.

### Description des modes de réalisation

Selon l'invention, le polyéthylène téréphtalate ou poly(téréphtalate d'éthylène), nommé encore simplement PET, a un motif élémentaire de répétition de formule :

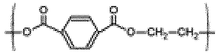

Classiquement, le PET est obtenu par polycondensation de l'acide téréphtalique (PTA), ou du diméthyle téréphtalate (DMT), avec l'éthylène glycol. Dans la suite du texte, l'expression « par mole de diester dans ladite charge polyester » correspond au nombre de moles de motif -[O-CO-O-(C₆H₄)-CO-O-CH₂-CH₂]-, qui est le motif diester issu de la réaction du PTA et de l'éthylène glycol, dans le PET compris dans ladite charge polyester.

Selon l'invention, le terme « monomère » ou « monomère diester » désigne avantageusement le bis(2-hydroxyéthyl) téréphtalate (BHET) de formule chimique HOC₂H₄-CO₂-(C₆H₄)-CO₂-C₂H₄OH, dans laquelle -(C₆H₄)- représente un cycle aromatique, et qui est le motif diester issu de la réaction du PTA et de l'éthylène glycol, dans le PET compris dans ladite charge polyester.

Le terme « oligomère » désigne typiquement un polymère de petite taille, constitué généralement de 2 à 20 motifs élémentaires de répétition. Selon l'invention, le terme « oligomère d'ester » ou « oligomère de BHET » désigne un oligomère d'ester téréphtalate, comprenant entre 2 et 20, de préférence entre 2 et 5, motifs élémentaires de répétition de formule -[O-CO-(C₆H₄)-CO-O-C₂H₄]-, avec -(C₆H₄)- un cycle aromatique.

Selon l'invention, les termes « diol » et « glycol » sont utilisés indifféremment et correspondent à des composés comprenant 2 groupements hydroxyle -OH. Le diol préféré est l'éthylène glycol, encore appelé mono-éthylène glycol ou MEG.

Ainsi, les flux diol ou effluent diol, mis en jeu dans les étapes du procédé de l'invention, comprennent ainsi de préférence de l'éthylène glycol (ou MEG) en quantité très majoritaire, c'est-à-dire de sorte que le MEG représente 95% poids ou plus du poids total dudit flux diol ou effluent diol.

Le terme « colorant » définit une substance soluble dans la matière polyester et utilisée pour la colorer. Le colorant peut être d'origine naturelle ou synthétique.

Selon l'invention, le terme « pigment », plus particulièrement pigment colorant et/ou opacifiant, définit une substance finement divisée, insoluble en particulier dans la matière polyester. Les pigments sont sous forme de particules solides, de taille comprise généralement entre 0,1 et 10 µm, et en majorité entre 0,4 et 0,8 µm. Ils sont souvent de nature minérale. Les pigments généralement utilisés, notamment pour opacifier, sont des oxydes métalliques comme TiO₂, CoAl₂O₄, Fe₂O₃, des silicates, des polysulfides, et du noir de carbone.

Selon la présente invention, les expressions « compris entre ... et ... » et « entre ... et ... » signifient que les valeurs limites de l'intervalle sont incluses dans la gamme de valeurs décrite. Si tel n'était pas le cas et que les valeurs limites n'étaient pas incluses dans la gamme décrite, une telle précision sera apportée par la présente invention.

Dans la suite, des modes de réalisation particuliers et/ou préférés de l'invention peuvent être décrits. Ils pourront être mis en œuvre séparément ou combinés entre eux sans limitation de combinaison lorsque cela est techniquement réalisable.

### Charge

Le procédé selon l'invention est alimenté par une charge polyester comprenant au moins un polyester, c'est-à-dire un polymère dont le motif de répétition de la chaine principale contient une fonction ester, et comprenant du polyéthylène téréphtalate (PET), de préférence comprenant au moins du PET opaque, et avantageusement du PET coloré, et de manière préférée du PET opaque et du PET coloré.

Ladite charge polyester est avantageusement une charge polyester à recycler, issue des filières de collecte et de tri des déchets, en particulier des déchets plastiques. Ladite charge polyester peut provenir, par exemple, de la collecte de bouteilles, barquettes, films, résines et/ou fibres constitués de polyéthylène téréphtalate.

Avantageusement, la charge polyester comprend au moins 50% poids, de préférence au moins 70% poids, de manière préférée au moins 90% poids de polyéthylène téréphtalate (PET).

De préférence, ladite charge polyester comprend au moins un PET choisi parmi le PET opaque, foncé ou coloré, multicouche et leurs mélanges. De manière très particulière, ladite charge polyester comprend au moins 10% en poids de PET opaque, de manière très préférée au moins 15% poids de PET opaque, ledit PET opaque étant avantageusement du PET opaque à recycler c'est-à-dire issu des filières de collecte et de tri.

Ladite charge polyester comprend avantageusement entre 0,1% et 10% poids de pigments, avantageusement entre 0,1 et 5% poids. Elle comprend également de préférence entre 0,05% et 1% de colorants, notamment entre 0,05 et 0,2% poids.

Dans les filières de collecte et de tri, les déchets polyester sont lavés et broyés avant de constituer la charge polyester du procédé selon l'invention.

La charge polyester peut être, en tout ou partie, sous forme de paillettes (ou flakes selon le terme anglais), dont la plus grande longueur est inférieure à 10 cm, préférentiellement comprise entre 5 et 25 mm ou sous forme de solide micronisé c'est-à-dire sous forme de particules de préférence ayant une taille comprise entre 10 micron et 1 mm. La charge peut également comprendre des impuretés « macroscopiques », de préférence moins de 5% poids, préférentiellement moins de 3% poids d'impuretés « macroscopiques », comme du verre, du métal, des plastiques autres que polyester (par exemple PP, PEHD...), du bois, du papier carton, des éléments minéraux. Ladite charge polyester peut également être, en tout ou partie, sous forme de fibres, telles que des fibres textiles, éventuellement prétraitées pour éliminer des fibres de coton, de polyamide, ou tout autre fibre textile autre que polyester, ou telles que des fibres de pneus, éventuellement prétraitées pour éliminer notamment des fibres polyamide ou des résidus de caoutchouc ou de polybutadiène. Ladite charge polyester peut, en outre, comprendre du polyester issu des rebuts de production des procédés de polymérisation et/ou de transformation de la matière polyester. La charge polyester peut également comprendre des éléments utilisés comme catalyseur de polymérisation et comme agents stabilisants dans les procédés de production de PET, tels que l'antimoine, le titane, l'étain.

### Étape a) de conditionnement

Ledit procédé selon l'invention comprend une étape a) de conditionnement qui comprend au moins une section de conditionnement alimentée au moins par ladite charge polyester, produisant un flux conditionné.

Ladite section de conditionnement de l'étape a) permet de chauffer et de mettre en pression ladite charge polyester aux conditions opératoires de l'étape b) de dépolymérisation.

Dans la section de conditionnement, la charge polyester est progressivement chauffée à une température proche voire légèrement supérieure à sa température de fusion de manière à devenir au moins en partie liquide. Avantageusement, au moins 70% poids de la charge polyester, très avantageusement au moins 80% poids, de préférence au moins 90% poids, préférentiellement au moins 95% poids de la charge polyester est sous forme liquide à l'issue de la section de conditionnement l'étape a). La température à laquelle la section de conditionnement de l'étape a) est mise en œuvre est avantageusement comprise entre 225 et 275°C. Cette température est maintenue la plus faible possible pour minimiser la dégradation thermique du polyester.

Selon un mode de réalisation préféré de l'invention, ladite section de conditionnement est une section d'extrusion qui correspond à une section de convoyage à vis. En d'autres termes, la section de conditionnement est opérée dans une extrudeuse.

Le temps de séjour dans ladite section d'extrusion, défini comme le volume de ladite section divisé par le débit volumique de charge est avantageusement inférieur à 15 min, de préférence inférieur à 10 min, et de manière préférée inférieure à 2 min. Avantageusement, la section d'extrusion permet de conditionner la charge polyester telle que le flux conditionné se trouve à une température entre 150-300°C, de préférence entre 225 et 275°C, et à une pression entre la pression atmosphérique (c'est-à-dire 0,1 MPa) et 20 MPa.

Ladite section d'extrusion est avantageusement connectée à un système d'extraction sous vide de manière à éliminer des impuretés telles que des gaz dissous, des composés organiques légers et/ou de l'humidité présents dans la charge. Ladite section d'extrusion peut également avantageusement comprendre un système de filtration pour éliminer des particules solides de taille supérieure à 40 µm, de préférence de taille inférieure à 2 cm, telles que des particules de sable.

Selon un mode de réalisation éventuel de l'invention, la section de conditionnement peut comprendre une zone de conditionnement, mise en œuvre avantageusement à une température entre 225 et 275°C, de préférence dans une extrudeuse, puis une phase de contact charge-diol dans laquelle la charge polyester est avantageusement mise en contact avec un flux diol, de préférence avec une fraction de l'effluent diol issu de l'étape c), de préférence de sorte que le nombre de moles de diol dudit flux diol, de préférence de ladite fraction de l'effluent diol issu de l'étape c), par mole de diester dans ladite charge polyester soit inférieur à 1,0, de manière préférée inférieur à 0,5, et avantageusement d'au moins 0,05. Cette mise en contact a pour effet d'initier la réaction de dépolymérisation de la charge polyester, avant l'introduction dans l'étape b) de dépolymérisation. Elle permet également de réduire la viscosité de la charge polyester, ce qui facilite son transport notamment vers l'étape b) de dépolymérisation. Ladite phase de contact charge-diol éventuelle peut être mise en œuvre dans un mélangeur statique ou dynamique. Lorsque la zone de conditionnement est mise en œuvre dans une extrudeuse, la phase de contact charge-diol peut être mise en œuvre au sein de l'extrudeuse ; dans ce cas, il s'agit d'une phase d'extrusion réactive. De préférence, le flux diol, en particulier la fraction de l'effluent diol issu de l'étape c), peut avantageusement être surchauffé préalablement à son alimentation dans la phase de contact charge-diol afin de faciliter la mise en température de la charge polyester.

La charge polyester peut également avantageusement être mélangée, avant ou dans la section de conditionnement, avec au moins une fraction de l'effluent impuretés lourdes issu de l'étape d), ladite fraction ayant préférentiellement été purifiée au préalable.

Le flux conditionné issu de la section de conditionnement est avantageusement envoyé vers l'étape b) de dépolymérisation.

### Étape b) de dépolymérisation

Le procédé selon l'invention comprend une étape de dépolymérisation par glycolyse alimentée au moins par le flux conditionné issu de l'étape a), et par un appoint de diol, opérée, en particulier en phase liquide, avantageusement à une température comprise entre 180 et 400°C, de préférence entre 200 et 300°C, de manière préférée entre 210°C et 280°C, à un temps de séjour dans ladite étape b) compris entre 0,1 et 10 h, de préférence entre 0,25 et 8 h, de manière préférée entre 0,5 et 6 h, et de sorte que la quantité totale de diol est ajustée entre 1 à 20 moles, de préférence entre 3 à 15 moles, de manière préférée entre 5 à 10 moles de diol par mole de diester de ladite charge polyester (correspondant un rapport pondéral de diol introduit par rapport au poids de la charge polyester compris entre 0,3 et 6,7, de préférence entre 1, 0 et 5,0, de manière préférée entre 1,7 et 3,3).

La pression d'opération de ladite étape b) est déterminée de manière à maintenir le système réactionnel en phase liquide. Cette pression est avantageusement d'au moins 0,1 MPa, préférentiellement au moins 0,4 MPa. Par système réactionnel, on entend l'ensemble des composants et phases présents au sein de ladite étape b).

Le temps de séjour est défini comme le rapport du volume de liquide de ladite section réactionnelle sur la somme du débit volumique de la charge polyester et de l'appoint de diol.

Le diol est avantageusement du monoéthylène glycol.

Ladite étape b) de dépolymérisation comprend avantageusement une ou plusieurs sections réactionnelles, de préférence au moins deux sections réactionnelles, de manière préférée entre 2 et 4 sections réactionnelles, fonctionnant en série. Chaque section réactionnelle peut être mise en œuvre dans tout type de réacteur connu de l'homme du métier permettant de réaliser une réaction de dépolymérisation ou de trans-estérification, de préférence, dans un réacteur agité par un système d'agitation mécanique ou/et par boucle de recirculation ou/et par fluidisation. Ledit réacteur peut comprendre un fond conique permettant de purger les impuretés. De manière préférée, ladite étape b) de dépolymérisation comprend au moins deux sections réactionnelles, de préférence entre 2 et 4 sections réactionnelles, fonctionnant en série, la(ou les)e section(s) réactionnelle(s) à partir de la deuxième section réactionnelle étant opérée à une température identique ou différente entre elles et inférieure ou égale à la température de la première section réactionnelle, de préférence inférieure, et préférentiellement inférieure de 10 à 50°C, voire inférieure de 20 à 40°C, par rapport à la température de la première section opérationnelle.

La réaction de glycolyse peut être réalisée en présence ou non d'un catalyseur.

Lorsque la réaction de glycolyse est réalisée en présence d'un catalyseur, ce dernier peut être homogène ou hétérogène et choisi parmi les catalyseurs d'estérification connus de l'homme du métier tels que les complexes oxydes et sels d'antimoine, d'étain, de titane, les alcoxydes de métaux des groupes (I) et (IV) de la classification périodique des éléments, les peroxydes organiques, les oxydes métalliques acido-basiques.

Un catalyseur hétérogène préféré comprend avantageusement au moins 50% masse par rapport à la masse totale du catalyseur, préférentiellement au moins 70% masse, avantageusement au moins 80% masse, très avantageusement au moins 90% masse, et façon encore plus avantageuse au moins 95% masse d'une solution solide constituée d'au moins une spinelle de formule ZₓAl₂O₍₃₊ₓ₎ dans laquelle x est compris entre 0 (borne exclue) et 1, et Z est choisi parmi Co, Fe, Mg, Mn, Ti, Zn, et comprenant au plus 50% masse d'alumine et d'oxyde de l'élément Z. Ledit catalyseur hétérogène préféré contient avantageusement au plus 10% masse de dopants choisis parmi le silicium, le phosphore et le bore pris seul ou en mélange. Par exemple, et de manière non limitative, ladite solution solide peut être constituée d'un mélange de spinelle ZnAl₂O₄ et de spinelle CoAl₂O₄, ou bien être constituée d'un mélange de spinelle ZnAl₂O₄, de spinelle MgAl₂O₄ et de spinelle FeAl₂O₄, ou bien être constituée uniquement de spinelle ZnAl₂O₄.

Le mode de réalisation particulier dans lequel ledit catalyseur hétérogène préféré est mis en œuvre a pour avantage de présenter une excellente conversion du PET par glycolyse en BHET. De plus, le catalyseur hétérogène de ce mode de réalisation particulier a pour propriété surprenante de capter les impuretés, en particulier les colorants, les additifs et les substances catalytiques utilisées pour la polymérisation et présentes dans le PET traité dans le procédé selon l'invention, telles que l'antimoine, le magnésium, le manganèse, le zinc, le titane, le phosphore, ce qui simplifie les étapes ultérieures de purification du BHET en vue de sa réutilisation dans un procédé de polymérisation.

De préférence, ladite étape de dépolymérisation est réalisée sans ajouter de catalyseur externe à la charge.

Ladite étape de dépolymérisation peut être avantageusement réalisée en présence d'un agent adsorbant solide sous forme de poudre ou mis en forme, dont la fonction est de capter au moins une partie des impuretés colorées, soulageant ainsi l'étape e) de décoloration. Ledit agent adsorbant solide est avantageusement un charbon actif.

La réaction de glycolyse permet de convertir la charge polyester en monomères et oligomères d'esters, avantageusement le PET en monomère Bis(2-Hydroxyethyl) téréphtalate (BHET) et oligomères de BHET. La conversion de la charge polyester dans ladite étape de dépolymérisation est supérieure à 50%, de préférence supérieure à 70%, de manière préférée supérieure à 85%. Le rendement molaire en BHET est supérieur à 50%, de préférence supérieur à 70%, de manière préférée supérieur à 85%. Le rendement molaire en BHET correspond au débit molaire de BHET en sortie de ladite étape b) sur le nombre de moles de diester dans la charge polyester alimentant ladite étape b).

Une boucle interne de recirculation est avantageusement mise en œuvre dans l'étape b), c'est-à-dire le soutirage d'une fraction du système réactionnel, la filtration de cette fraction, et la réinjection de ladite fraction dans ladite étape b). Cette boucle interne permet d'éliminer les impuretés solides, « macroscopiques », éventuellement comprises dans le liquide réactionnel.

Avantageusement, l'étape b) de dépolymérisation permet d'obtenir un effluent réactionnel qui est envoyé vers une étape c) de séparation du diol.

### Étape c) de séparation du diol

Le procédé selon l'invention comprend une étape c) de séparation du diol, alimentée au moins par l'effluent de l'étape b), opérée à une température comprise entre 100 et 250°C, à une pression inférieure à celle de l'étape b) et produisant un effluent diol et un effluent riche en monomères liquide.

L'étape c) a pour fonction principale de récupérer tout ou partie du diol non réagi.

L'étape c) est opérée à une pression inférieure à celle de l'étape b) de manière à vaporiser une fraction de l'effluent de l'étape b) en un effluent gaz et un effluent liquide. Ledit effluent liquide constitue l'effluent riche en monomères liquides. L'effluent gaz, constitué à plus de 50% poids de diol, de préférence plus de 70% poids, de manière préférée plus de 90% poids, constitue un effluent diol.

L'étape c) est avantageusement mise en œuvre dans une de section de séparation gaz-liquide ou une succession de sections de séparation gaz-liquide, avantageusement de 2 à 5 sections de séparation successives, très avantageusement de 3 à 5 séparations successives. Chacune des sections de séparation gaz-liquide produit un effluent liquide et un effluent gaz. L'effluent liquide de la section antérieure alimente la section ultérieure. L'ensemble des effluents gaz est récupéré pour constituer l'effluent diol. L'effluent liquide issu de la dernière section de séparation gaz-liquide constitue l'effluent riche en monomères liquide.

Avantageusement, au moins une des sections de séparation gaz-liquide peut être mise en œuvre dans un évaporateur à film tombant ou un évaporateur à film raclé ou une distillation court-trajet.

L'étape c) est opérée de telle sorte que la température des effluents liquides soit maintenue au-dessus de la valeur en dessous de laquelle le monomère de polyester précipite, et en dessous d'une valeur haute, dépendant du ratio molaire diol/monomère, au-dessus de laquelle le monomère se re-polymérise de manière significative. La température dans l'étape c) est comprise entre 100 et 250°C, de préférence entre 110 et 220°C, de manière préférée entre 120 et 210°C. L'opération en une succession de séparations gaz-liquide, avantageusement en une succession de 2 à 5, préférentiellement de 3 à 5 séparations successives, est particulièrement avantageuse car elle permet d'ajuster dans chaque séparation la température de l'effluent liquide répondant aux contraintes précitées.

La pression dans l'étape c) est ajustée pour permettre l'évaporation du diol à une température tout en minimisant la re-polymérisation et permettant une intégration énergétique optimale. Elle est généralement comprise entre 0,00001 et 0,2 MPa, de préférence entre 0,00004 et 0,15 MPa, de manière préférée entre 0,00004 et 0,1 MPa.

La ou les section(s) de séparation sont avantageusement agitée(s) par toute méthode connue de l'homme du métier.

L'effluent diol peut contenir d'autres composés comme des colorants, des alcools légers, de l'eau, du diéthylène glycol. Au moins une fraction de l'effluent diol peut avantageusement être recyclé, sous forme liquide, c'est-à-dire après condensation, vers l'étape a) et/ou l'étape b) et/ou l'étape e), éventuellement en mélange avec un apport en diol externe au procédé selon l'invention.

Tout ou partie dudit effluent diol peut être traité dans une étape de purification préalablement à son recyclage. Cette étape de purification peut comprendre, de manière non exhaustive, une adsorption sur solide (par exemple sur charbon actif) pour éliminer les colorants et une ou plusieurs distillations pour séparer les impuretés comme le diéthylène glycol, l'eau et d'autres alcools.

### Étape d) de séparation du monomère

Le procédé selon l'invention comprend une étape d) de séparation de l'effluent riche en monomères issu de l'étape c) en un effluent impuretés lourdes et un effluent monomères pré-purifié.

Ladite étape d) est avantageusement opérée à une température inférieure à 250°C, de manière préférée inférieure à 230°C, et de façon très préférée inférieure à 200°C, et de préférence supérieure à 110°C, et une pression inférieure à 0,001 MPa, de préférence inférieure à 0,0005 MPa, de préférence supérieure à 0,000001 MPa, avec un temps de séjour liquide inférieur à 10 min, de préférence inférieur à 5 min, de manière préférée inférieur à 1 min, et de préférence supérieur à 0,1 seconde.

Cette étape d) de séparation a pour objectif de séparer le monomère, en particulier le BHET, qui est vaporisé, des oligomères, non convertis entièrement, qui restent liquides et captent donc également les impuretés lourdes, notamment les pigments, du polymère polyester non converti, d'autres polymères éventuellement présents et des catalyseurs de polymérisation, tout en minimisant la perte en monomères par re-polymérisation. Quelques oligomères peuvent être éventuellement entrainés avec le monomère, en particulier ceux de petite taille. Ces impuretés lourdes se retrouvent avec les oligomères dans l'effluent impuretés lourdes.

Du fait de la présence possible dans la charge polyester de catalyseurs de polymérisation, la séparation doit être réalisée avec des temps de séjour liquide très courts et à une température n'excédant pas 250°C, afin de limiter tout risque de re-polymérisation du monomère lors de cette étape. Une séparation par distillation atmosphérique simple n'est donc pas envisageable.

L'étape d) de séparation est avantageusement mise en œuvre dans une section de séparation comprenant un système d'évaporation à film tombant ou film raclé ou par distillation à court trajet à film tombant ou à film raclé, en particulier par distillation à court trajet à film tombant ou à film raclé.

Une pression opératoire très faible est nécessaire pour pouvoir opérer l'étape d) à une température inférieure à 250°C, de préférence inférieure à 230°C, tout en permettant la vaporisation du monomère.

Un inhibiteur de polymérisation peut avantageusement être mélangé à l'effluent riche en monomères liquide avant d'alimenter ladite étape d).

Un fluxant peut également être avantageusement mélangé à l'effluent riche en monomères liquide avant d'alimenter ladite étape d),de manière à faciliter l'élimination des impuretés lourdes, notamment des pigments, en fond du système d'évaporation ou de distillation court trajet. Ce fluxant doit avoir une température d'ébullition très supérieure au monomère, en particulier au BHET, dans les conditions d'opération de l'étape d). Il peut s'agir par exemple de polyéthylène glycol, ou d'oligomères du PET.

Ledit effluent impuretés lourdes comprend en particulier les pigments, des oligomères et éventuellement du BHET non séparé. Ledit effluent impuretés lourdes est avantageusement recyclé, en tout ou partie, vers l'étape a) de conditionnement. Ledit effluent impuretés lourdes peut subir avantageusement au moins une étape de purification, de manière préférée une étape de filtration préalablement à son recyclage de manière à réduire la quantité de pigments et/ou autres impuretés solides. La partie dudit effluent impuretés lourdes séparées et à forte teneur en pigments peut avantageusement être purgée du procédé et envoyée vers un système d'incinération.

Ledit effluent monomères pré-purifié issu de la section de séparation de l'étape d) est avantageusement envoyé vers l'étape e).

Optionnellement, ledit effluent monomères pré-purifié issu de la section de séparation de l'étape d) peut être envoyé dans une section de séparation gaz-liquide, opérée dans tout équipement connu de l'homme du métier, à une température comprise entre 100 et 250°C, de préférence entre 110 et 200°C, et de manière préférée entre 120 et 180°C, et à une pression comprise entre 0,00001 et 0,1 MPa, de préférence entre 0,00001 et 0,01 MPa, et de manière préférée entre 0,00001 et 0,001 MPa. Ladite section de séparation gaz-liquide optionnelle permet de séparer un effluent diol gazeux et un effluent monomère pré-purifié liquide. Ladite séparation gaz-liquide permet de réduire encore la quantité de diol restant dans l'effluent monomères pré-purifié, voire éliminer le diol résiduel, en récupérant dans ledit effluent diol gazeux plus de 50% pds, de préférence plus 70% pds, de manière préférée plus de 90% pds du diol éventuellement entrainé dans l'étape d) avec l'effluent monomère pré-purifié. La quantité de monomère entrainé dans ledit effluent diol gazeux est de préférence inférieure à 1% pds, de manière préférée inférieure à 0,1% pds et de manière plus préférée inférieure à 0,01% pds de la quantité de monomère présent dans l'effluent monomère pré-purifié. Ledit effluent diol gazeux est ensuite avantageusement condensé, éventuellement prétraité dans une étape de purification et recyclé avec l'effluent diol issu de l'étape c) vers l'étape a) et/ou l'étape b) et/ou en mélange dans l'étape e). Dans le cas où le procédé comprend cette section de séparation gaz-liquide, c'est l'effluent monomères pré-purifié liquide obtenu à l'issue de ladite section gaz-liquide optionnelle qui est envoyé vers l'étape e).

### Étape e) de décoloration

Le procédé selon l'invention comprend une étape de décoloration de l'effluent monomères pré-purifié issu de l'étape d), ou optionnellement l'effluent monomère pré-purifié liquide, produisant un effluent monomères purifié.

Ladite étape e) permet avantageusement d'éliminer les colorants résiduels de l'effluent monomères pré-purifié, en particulier les colorants dont le point d'ébullition est inférieur au point de coupe, c'est-à-dire aux conditions de température et pression mises en œuvre notamment dans l'étape d) de séparation du monomère. En effet, ces derniers, entrainés avec l'effluent monomères pré-purifié qu'ils colorent, peuvent ainsi être éliminés de manière efficace par adsorption dans ladite étape e).

Avantageusement, ladite étape e) met en œuvre au moins une section de mélange de l'effluent monomères pré-purifié issu de l'étape d), ou optionnellement l'effluent monomère pré-purifié liquide, avec un solvant, et une section d'adsorption.

Ladite section de mélange est alimentée par l'effluent monomères liquide pré-purifié issu de l'étape d), ou optionnellement l'effluent monomère pré-purifié liquide, et un solvant, de préférence choisi parmi les diols, par exemple l'éthylène glycol. De préférence, ledit solvant comprend, de préférence consiste en, une fraction de l'effluent diol issu de l'étape c), un appoint en diol externe au procédé selon l'invention, ou leurs mélanges. De manière très avantageuse, ledit solvant est une fraction de l'effluent diol issu de l'étape c).

De préférence, la quantité de solvant introduit dans la section de mélange est ajustée de sorte que l'effluent monomères pré-purifié, ou optionnellement l'effluent monomère pré-purifié liquide, représente entre 20 et 90% poids, préférentiellement entre 30 et 80% poids, de manière préférée entre 50 et 75% poids, du poids total du mélange de ladite section de mélange. Ladite section de mélange est avantageusement opérée à une température comprise entre 100 et 250°C, de préférence entre 110 et 200°C, et de manière préférée entre 120 et 180°C, et à une pression comprise entre 0,1 et 1,0 MPa, de préférence entre 0,1 et 0,8 MPa, et de manière préférée entre 0,2 et 0,5 MPa. Le solvant peut être chauffé, préalablement à ladite section de mélange, de préférence à la température à laquelle la section de mélange est opérée, en particulier à une température entre 100 et 250°C, de préférence entre 110 et 200°C, et de manière préférée entre 120 et 180°C.

De préférence, la section de mélange met en œuvre un mélangeur statique ou dynamique, de manière préférée un mélangeur statique. Le temps de séjour dans la section de mélange, défini comme le volume total de liquide dans ladite section de mélange par rapport au débit de mélange liquide sortant de ladite section de mélange, est avantageusement compris entre 0,5 seconde et 20 minutes, de préférence entre 1 seconde et 5 minutes, de manière préférée entre 3 secondes et 1 minutes.

Ledit mélange de la section de mélange alimente avantageusement la section d'adsorption. La section d'adsorption est opérée en présence d'au moins un adsorbant, et de préférence jusqu'à cinq adsorbants différents, à une température comprise entre 100 et 200°C, de préférence entre 100 et 170°C, et de manière préférée entre 120 et 150°C, et préférentiellement à une pression entre 0,1 et 1,0 MPa, en particulier entre 0,1 et 0,8 MPa et plus particulièrement entre 0,2 et 0,5 MPa.

La section d'adsorption comprend avantageusement au moins un adsorbeur. Très avantageusement, le temps de séjour du mélange dans chaque adsorbeur de la section d'adsorption est compris entre 20 minutes et 40 heures, de préférence entre 1 heure et 30 heures, de préférence entre 1 heure et 20 heures. Le temps de séjour est défini comme le ratio du volume interne de l'adsorbeur sur le débit volumique du mélange issu de la section de mélange.

De préférence, la section d'adsorption est opérée en présence d'un adsorbant ou de deux adsorbants différents. Selon l'invention, des adsorbants sont dits différents lorsque leur nature est différente et/ou leur composition et/ou leur granulométrie différente et/ou leurs caractéristiques texturales, comme le volume poreux. De manière plus préférée, les adsorbants différents sont de nature différente.

Lorsque la section d'adsorption comprend deux ou plus adsorbants différents, lesdits adsorbants différents sont en mélange ou placés en série dans ladite section d'adsorption, de préférence en série et plus préférentiellement chacun des adsorbants étant dans des adsorbeurs (par exemple réacteurs ou colonnes) différents placés en série. Il peut en effet être avantageux d'associer plusieurs adsorbants différents, en particulier de nature différente, afin d'optimiser l'élimination des colorants résiduels qui peuvent être eux aussi de nature très différente. En effet, la charge polyester du procédé étant issue de déchets polyester, comme des déchets d'emballages en PET ou de bouteilles plastiques, elle peut comprendre un très grand nombre de PET colorés et/ou opaques et donc un très grand nombre de composés colorants différents. La coloration de l'effluent issu de l'étape d) peut également provenir d'une dégradation ou transformation de composés constituant la charge lors des étapes de conditionnement a), de polymérisation b), de séparation du diol c) et de séparation du monomère d).

Avantageusement, ledit (ou lesdits) adsorbant(s) est(sont) choisi(s) parmi les charbons actifs, les alumines et les argiles. Les charbons actifs qui peuvent être utilisés sont par exemple issus du petcoke, de la houille ou de toute autre origine fossile, ou issus de la biomasse comme le bois, la noix de coco ou toute autre source de biomasse. Différentes matières premières peuvent également être mélangées pour obtenir des charbons actifs qui pourront éventuellement être utilisés comme adsorbants dans ladite section d'adsorption. Les argiles peuvent être par exemple des hydroxydes doubles lamellaires ou des argiles naturelles ou transformées telles que celles connues de l'homme du hétier sous le terme de terres décolorantes. De manière préférée, au moins un adsorbant est un charbon actif. Ainsi, lorsque la section d'adsorption comprend un seul type d'adsorbant, ledit adsorbant est un charbon actif et, lorsque la section d'adsorption comprend deux ou plus adsorbants différents, un adsorbant est un charbon actif et le(ou les) autre(s) est(sont) un autre charbon actif, une alumine ou une argile, de préférence un charbon actif ou une argile, plus particulièrement une argile.

La section d'adsorption est mise avantageusement en œuvre en mode lit fixe traversé, c'est-à-dire dans au moins un adsorbeur à lit fixe d'adsorbant(s), en particulier au moins une colonne d'adsorbant(s), pouvant fonctionner en mode ascendant ou descendant, de manière préférée en mode ascendant, ou dans au moins un réacteur agité en continu, appelé encore « Continuous Stirring Tank Reactor » (CSTR) selon le terme anglo-saxon. Dans le cas où la section est mise en œuvre dans au moins un réacteur agité de type CSTR, le ou les réacteur(s) est(sont) suivi(s) d'un système de filtration pour récupérer ledit (ou lesdits) adsorbant(s) qui est(sont) en suspension dans le liquide traité. De manière préférée, la section d'adsorption est mise en œuvre en mode lit fixe traversé.

De préférence, chaque adsorbant de la section d'adsorption a un volume poreux (Vp), déterminé par porosimétrie au mercure, supérieur ou égal à 0,25 ml/g, préférentiellement supérieur ou égal à 0,40 ml/g, de manière préférée supérieur ou égal à 0,50 ml/g, et de préférence inférieur ou égal à 5 ml/g.

Dans le cas où la section d'adsorption comprend au moins deux adsorbants différents, alors les adsorbants peuvent :
- soit être tous présents dans chaque colonne de ladite section d'adsorption, en mélange ou dans des lits successifs,
- soit être utilisés chacun dans une sous-section d'adsorption, les sous-sections étant placées en série les unes par rapport aux autres, chaque sous-section d'adsorption étant constituée de plusieurs, de préférence entre 2 et 4, colonnes d'adsorbant en lit fixe.

Très avantageusement, ladite section d'adsorption ou chacune des sous-sections de ladite section d'adsorption comprend plusieurs colonnes d'adsorbant en lit fixe, en particulier au moins deux colonnes d'adsorbant, de préférence entre 2 et 4 colonnes du même adsorbant, en particulier deux colonnes du même adsorbant. Lorsque la section d'adsorption ou la sous-section d'adsorption comprend deux colonnes du même adsorbant, un mode de fonctionnement peut être un fonctionnement appelé « swing », selon le terme anglo-saxon consacré, dans lequel l'une des colonnes est en ligne tandis que l'autre colonne est en réserve. Lorsque l'adsorbant de la colonne en ligne est usé, cette colonne est isolée tandis que la colonne en réserve est mise en ligne. L'adsorbant usé peut être ensuite régénéré in-situ et/ou remplacé par de l'adsorbant frais pour à nouveau être remis en ligne une fois que l'autre colonne a été isolée. Un autre mode de fonctionnement des colonnes d'adsorbants est d'avoir au moins deux colonnes fonctionnant en série ; lorsque l'adsorbant de la colonne en tête (c'est-à-dire la première colonne de la série) est usé, cette première colonne est isolée et l'adsorbant usée est soit régénéré in-situ ou remplacé par de l'adsorbant frais. La colonne est ensuite remise en ligne en dernière position dans la série de colonne et ainsi de suite. Ce fonctionnement est appelé « lead-lag », selon le terme anglo-saxon consacré. De manière très préférée, la section d'adsorption est mise en œuvre dans au moins deux colonnes du même adsorbant, de préférence dans 2 à 4 colonnes du même adsorbant, préférentiellement dans deux colonnes du même adsorbant, fonctionnant en « lead-lag ».

Dans un mode de réalisation très particulier de l'invention dans lequel la section d'adsorption comprend deux adsorbants différents, la section d'adsorption comprend très préférentiellement une première sous-section comprenant au moins deux, de préférence entre 2 et 4, colonnes à lit fixe de charbon actif fonctionnant de préférence en swing ou en lead-lag et une deuxième sous-section comprenant au moins deux, de préférence entre 2 et 4, colonnes d'un autre adsorbant, de manière préférée choisi parmi un autre charbon actif ou une argile, fonctionnant en swing ou en lead-lag et placée en amont ou en aval de la première sous-section de colonnes de charbon actif en lit fixe.

L'association de au moins deux colonnes du même adsorbant permet de palier en particulier au colmatage éventuellement rapide de l'adsorbant, dû notamment à la présence des colorants dans l'effluent monomères pré-traité. La présence d'au moins deux colonnes d'adsorbant facilite en effet le remplacement et/ou la régénération de l'adsorbant, avantageusement sans arrêt de l'unité de décoloration, voire du procédé, permettant ainsi de diminuer les risques de colmatage et donc d'éviter l'arrêt de l'unité dû au colmatage, de maitriser les coûts et de limiter la consommation d'adsorbant, tout en assurant une production en continue de monomères diester purifié. Cette association d'au moins deux colonnes au moins du même adsorbant, en particulier fonctionnant en « lead-lag » permet également de maximiser la capacité d'adsorption dudit adsorbant.

De préférence, chaque adsorbant est sous forme de granulés, d'extrudés ou de poudre. De manière préférée, chaque adsorbant est sous forme de granulés ou d'extrudés lorsque la section d'adsorption est mise en œuvre en mode lit fixe traversé, et il est sous forme de poudre lorsque la section d'adsorption est mise en œuvre en réacteur agité de type CSTR. La taille dudit au moins adsorbant, en particulier lorsqu'il est sous forme de granulés ou d'extrudés, est telle que la plus petite dimension dudit au moins adsorbant (correspondant au diamètre du cercle circonscrit au motif des granulés ou extrudés polylobiques ou au diamètre du cylindre circonscrit au motif cylindrique des extrudés de type cylindrique ; cette dimension est encore appelée « diamètre ») est de préférence comprise entre 0,1 et 5 mm, préférentiellement entre 0,3 et 2 mm. Par exemple, les extrudés de charbon actif de diamètre 0,8 mm commercialisés par la société Cabot Norit ou les granulés compris dans la gamme de taille entre 0,4 et 1,7 mm commercialisés par la société Chemviron peuvent convenir comme adsorbant dans la section d'adsorption de l'étape e) de décoloration.

L'étape e) de décoloration peut également comprendre avantageusement une phase de régénération dudit (ou desdits) adsorbant(s).

L'effluent monomères purifié obtenu à l'issue de l'étape e) du procédé de l'invention est pratiquement incolore voire incolore, à l'œil. De préférence, l'effluent monomères purifié est caractérisé par spectrométrie UV-visible afin d'identifier la présence de bandes d'absorption dans le domaine du visible, c'est-à-dire entre 400 et 800 nm. De préférence, l'effluent monomères purifié est caractérisé selon cette méthode, c'est-à-dire par spectrométrie UV-visible, entre 400 et 800 nm, à une température supérieure au point de fusion de l'effluent caractérisé, c'est-à-dire en milieu liquide, soit de préférence entre 120°C et 150°C, et avec un trajet optique de 5 mm. L'effluent monomères purifié obtenu selon l'invention présente un spectre ne montrant aucune bande d'absorption significative (c'est-à-dire ne pouvant pas être différenciée du bruit de fond) dans la gamme des longueurs d'onde visible (c'est-à-dire entre 400 et 800 nm).

L'effluent monomère purifié alimente avantageusement une étape de polymérisation connue de l'homme du métier en vue de produire du PET que rien ne distingue du PET vierge, avantageusement en aval de l'alimentation en éthylène glycol, en acide téréphtalique ou en diméthyltéréphtalate suivant l'étape de polymérisation retenue. L'alimentation de l'effluent monomère purifié dans une étape de polymérisation permet de diminuer d'un débit équivalent l'alimentation en diméthyltéréphtalate ou en acide téréphtalique.

Les exemples suivants illustrent l'invention sans en limiter la portée.

### Exemples

Dans l'exemple suivant, les étapes a) de conditionnement, b) de dépolymérisation, c) de séparation du diol et d) de séparation du monomère sont identiques et décrites ci-dessous.

Une charge polyester comprenant en particulier 20% poids de PET opaque est issue des filières de collecte et de tri pour être traitée.

4 kg/h de paillettes de ladite charge polyester comprenant 20% poids de PET opaque qui contient lui-même 6,2% poids de pigment TiO₂, et 11,5 kg/h d'éthylène glycol (MEG), sont portés à une température de 250°C, injectés dans un premier réacteur agité maintenu à 250°C puis dans un second et un troisième réacteur agité maintenus à 220°C. Les réacteurs sont maintenus à une pression de 0,4 MPa. Le temps de séjour, défini comme le rapport du volume liquide dans le réacteur par la somme des débits volumiques liquides entrant dans le réacteur, est fixé à 20 min dans le premier réacteur et 2,1 h dans le second et troisième réacteur. A la sortie du troisième réacteur, l'effluent réactionnel est constitué de 67,7 % poids de diol composé très majoritairement de MEG (comprenant 95% poids de MEG ou plus), 25,8 % poids de monomère diester composé très majoritairement de BHET (comprenant 95% poids de BHET ou plus), 0,32 % poids de TiO₂, et 6,1 % poids de composés lourds contenant entre autres des dimères et/ou oligomères de BHET.

L'éthylène glycol présent dans l'effluent réactionnel est séparé par évaporation dans une succession de 2 ballons flash à des températures allant de 180°C à 120°C et des pressions de 0,04 MPa à 0,004 MPa et d'un évaporateur à film raclé opéré à 175°C et 0.0005 MPa. A l'issue de cette étape d'évaporation, un flux riche en MEG de 10,46 kg/h et un flux liquide riche en BHET de 5,02 kg/h sont récupérés. Le flux riche en MEG est constitué très majoritairement d'éthylène glycol et peut ainsi être pour une part recyclé vers le réacteur de dépolymérisation et pour une autre part envoyé vers l'étape de décoloration si nécessaire ou une étape de purification préalablement à l'étape de décoloration. Le flux liquide riche en BHET est constitué de 79,6% poids de monomère diester, 0,6 % poids de MEG et 1,0 % pods de TiO₂ et 18,8 % poids de composés lourds contenant entre autres des dimères de BHET.

Le flux liquide riche en BHET est ensuite injecté dans un évaporateur court trajet, autrement appelé distillation court-trajet ou short-path distillation en dénomination anglophone, opérée à une pression de 20 Pa. Une huile chaude à 215°C permet l'évaporation du BHET qui est ensuite condensé dans l'évaporateur court-trajet à 130°C pour donner un flux liquide de BHET pré-purifié. Le temps de séjour dans l'évaporateur court-trajet est de 1 min. Le flux liquide de BHET pré-purifié représente un débit de 3,8 kg/h et est récupéré comme distillat de l'évaporateur court-trajet. Il est constitué de 99 % poids de monomère diester et est exempt de trace de TiO₂. Un résidu lourd avec un débit de 1,19 kg/h est récupéré comme résidu de l'évaporateur court-trajet et est constitué de 16,7 % poids de monomère diester, 79,2% poids d'oligomères de BHET et 4,1 % poids de TiO₂.

L'effluent gaz est condensé à 130°C pour donner un flux liquide de BHET pré-purifié.

### EXEMPLE 1 - CONFORME

Le flux liquide de BHET pré-purifié et contenant 99% poids de diester BHET est comprimé jusqu'à 0,15 MPa et alimente une section de mélange qui est alimentée également en une fraction du flux de MEG issue de l'étape de vaporisation. Le débit d'alimentation en ladite fraction du flux de MEG est ajusté de sorte que ledit flux liquide de BHET représente 50% poids du mélange final. Ladite section est opérée à 150°C, à une pression de 0,15 MPa.

Le mélange obtenu alimente ensuite une section d'adsorption constituée de deux colonnes chacune remplie d'un adsorbant. La section d'adsorption est opérée à 150°C, à une pression de 0,15 MPa. Une colonne est mise sous flux, l'autre restant en réserve. L'adsorbant utilisé pour le remplissage des deux colonnes est un charbon actif constitué d'extrudés cylindriques de 0.8 mm de diamètre, référence ROY 0.8 de la société Cabot Norit.

Le temps de séjour est fixé à 3 h.

La coloration de l'effluent en sortie de colonne est suivie par spectrométrie UV-visible in situ, à l'aide d'une sonde UV-visible de la marque Hellma, série Falcata, trajet optique 5 mm. La coloration est suivie entre 400 et 800 nm, via l'apparition d'une bande d'absorption significative sur cette plage de longueur d'onde.

L'apparition d'une coloration, identifiée via l'apparition d'une bande d'absorption autour de 450 nm, est observée au bout de 7 jours. Ce temps définit la durée de fonctionnement de la colonne avant de passer le flux à traiter sur la colonne en réserve.

La colonne contenant l'adsorbant usée est soit déchargée, soit régénérée avant d'être remise en fonctionnement sur le flux une fois la colonne en réserve usée.

## Revendications

1. Procédé de dépolymérisation d'une charge polyester comprenant du polyéthylène téréphtalate (PET) opaque, ledit procédé comprenant :
a) une étape de conditionnement comprenant une section de conditionnement alimentée au moins par ladite charge polyester, pour produire un flux conditionné ;
b) une étape de dépolymérisation par glycolyse alimentée au moins par le flux conditionné et par un appoint de diol de sorte que la quantité molaire de diol est ajustée entre 1 à 20 moles de diol par mole de diester de ladite charge polyester, opérée à une température comprise entre 180 et 400°C, et un temps de séjour compris entre 0,1 et 10 h ;
c) une étape de séparation du diol alimentée au moins par l'effluent de l'étape b), opérée à une température comprise entre 100 et 250°C, à une pression inférieure à celle de l'étape b) et produisant un effluent diol et un effluent riche en monomères liquide, ladite étape de séparation du diol étant mise en œuvre dans 1 à 5 sections de séparation gaz-liquide successives produisant chacune un effluent gaz et un effluent liquide, l'effluent liquide de la section antérieure alimentant la section ultérieure, l'effluent liquide issu de la dernière section de séparation gaz-liquide constituant l'effluent riche en monomères liquide, l'ensemble des effluents gaz étant récupéré pour constituer l'effluent diol ;
d) une étape de séparation de l'effluent riche en monomères liquides issu de l'étape c) en un effluent impuretés lourdes et un effluent monomères pré-purifié, opérée à une température inférieure à 250°C et une pression inférieure à 0,001 MPa avec un temps de séjour liquide inférieur à 10 min, et
e) une étape de décoloration de l'effluent monomères pré-purifié, comprenant au moins une section de mélange de l'effluent monomères pré-purifié issu de l'étape d) avec un solvant de sorte que l'effluent monomères pré-purifié représente entre 20 et 90% poids du poids total du mélange, et une section d'adsorption opérée à une température comprise entre 100 et 200°C, et à une pression comprise entre 0,1 et 1,0 MPa, en présence d'au moins un adsorbant choisi parmi les charbons actifs, les alumines et les argiles, pour produire un effluent monomères purifié.

2. Procédé selon la revendication 1 dans lequel ladite charge polyester comprend au moins 50% poids, de préférence au moins 70% poids, de manière préférée au moins 90% poids de polyéthylène téréphtalate.

3. Procédé selon la revendication 1 ou 2 dans lequel ladite charge polyester comprend au moins 10% en poids, de préférence au moins 15% poids, de PET opaque.

4. Procédé selon l'une des revendications précédentes dans lequel ladite charge polyester comprend du PET coloré.

5. Procédé selon l'une des revendications précédentes dans lequel ladite charge polyester comprend entre 0,1% et 10% poids de pigments, de préférence entre 0,1 et 5% poids de pigments.

6. Procédé selon l'une des revendications précédentes dans lequel ladite charge polyester comprend entre 0,05% et 1% de colorants, notamment entre 0,05 et 0,2% poids de colorants.

7. Procédé selon l'une des revendications précédentes dans lequel le solvant dans l'étape e) de décoloration comprend une fraction de l'effluent diol issu de l'étape c), un appoint en diol externe au procédé selon l'invention, ou leurs mélanges.

8. Procédé selon l'une des revendications précédentes dans lequel l'effluent monomères pré-purifié représente entre 30 et 80% poids, de manière préférée entre 50 et 75% poids, du poids total du mélange dans l'étape e).

9. Procédé selon l'une des revendications précédentes dans lequel au moins un adsorbant dans l'étape e) est un charbon actif.

10. Procédé selon l'une des revendications précédentes dans lequel la section d'adsorption dans l'étape e) est opérée en présence d'un adsorbant ou de deux adsorbants différents.

11. Procédé selon l'une des revendications précédentes dans lequel la section d'adsorption comprend deux ou plus adsorbants différents, lesdits adsorbants différents étant en mélange ou placés en série dans ladite section d'adsorption.

12. Procédé selon la revendication précédente dans lequel chacun desdits adsorbants différents sont dans des réacteurs différents placés en série.

13. Procédé selon l'une des revendications précédentes dans lequel la section d'adsorption de l'étape e) est mise en œuvre en mode lit fixe d'adsorption.

14. Procédé selon l'une des revendications précédentes dans lequel la section d'adsorption de l'étape e) est opérée à une température comprise entre 100 et 170°C, de préférence entre 120°C et 150°C.

## Patentansprüche

1. Verfahren zur Depolymerisierung eines Polyester-Einsatzmaterials, das opakes Polyethylenterephthalat (PET) umfasst, das Verfahren umfassend:
a) einen Schritt zur Aufbereitung, der einen Aufbereitungsabschnitt umfasst, dem mindestens das Polyester-Einsatzmaterial zugeführt wird, um einen aufbereiteten Strom zu erzeugen;
b) einen Schritt zur Depolymerisierung durch Glykolyse, dem mindestens der aufbereitete Strom und eine Diol-Zugabe zugeführt werden, so dass die Molmenge an Diol auf 1 bis 20 Mol Diol je Mol Diester des Polyester-Einsatzmaterials eingestellt wird, und der bei einer Temperatur zwischen 180 °C und 400 °C und mit einer Verweilzeit zwischen 0,1 und 10 Stunden durchgeführt wird;
c) einen Schritt des Trennens des mindestens durch den Abstrom des Schritts b) zugeführten Diols, der bei einer Temperatur zwischen 100 und 250 °C bei einem Druck unter dem des Schritts b) durchgeführt wird und einen Diol-Abstrom und einen flüssigen monomerreichen Abstrom erzeugt, wobei der Schritt des Trennens des Diols in 1 bis 5 aufeinander folgenden Abschnitten zur Gas-Flüssigkeits-Trennung durchgeführt wird, die jeweils einen Gasabstrom und einen flüssigen Abstrom erzeugen, wobei der flüssige Abstrom des vorhergehenden Abschnitts dem nachfolgenden Abschnitt zugeführt wird, wobei der aus dem letzten Abschnitt zur Gas-Flüssigkeits-Trennung hervorgegangene Abstrom den flüssigen monomerreichen Abstrom bildet, wobei die Gesamtheit der Gasabströme zurückgewonnen wird, um den Diol-Abstrom zu bilden;
d) einen Schritt der Trennens des aus dem Schritt c) hervorgegangenen flüssigen monomerreichen Abstroms in einen Abstrom mit schweren Verunreinigungen und einen vorgereinigten Monomer-Abstrom, der bei einer Temperatur unter 250 °C und einem Druck unter 0,001 MPa mit einer Flüssig-Verweilzeit von weniger als 10 min durchgeführt wird, und
e) einen Schritt des Entfärbens des vorgereinigten Monomer-Abstroms, umfassend mindestens einen Abschnitt zum Mischen des aus dem Schritt d) hervorgegangenen vorgereinigten Monomer-Abstroms mit einem Lösungsmittel, so dass der vorgereinigte Monomer-Abstrom zwischen 20 und 90 Gew.-% des Gesamtgewichts der Mischung ausmacht, und einen Adsorptionsabschnitt, der bei einer Temperatur zwischen 100 und 200 °C und bei einem Druck zwischen 0,1 und 1,0 MPa in Gegenwart mindestens eines Adsorbens, das aus Aktivkohlen, Aluminiumoxiden und Tonerden ausgewählt ist, betrieben wird, um einen gereinigten Monomer-Abstrom zu erzeugen.

2. Verfahren nach Anspruch 1, wobei das Polyester-Einsatzmaterial mindestens 50 Gew.-%, vorzugsweise mindestens 70 Gew.-%, bevorzugt mindestens 90 Gew.-% Polyethylenterephthalat umfasst.

3. Verfahren nach Anspruch 1 oder 2, wobei das Polyester-Einsatzmaterial mindestens 10 Gew.-%, vorzugsweise mindestens 15 Gew.-% opakes PET umfasst.

4. Verfahren nach einem der vorhergehenden Ansprüche, wobei das Polyester-Einsatzmaterial farbiges PET umfasst.

5. Verfahren nach einem der vorhergehenden Ansprüche, wobei das Polyester-Einsatzmaterial zwischen 0,1 und 10 Gew.-% Pigmente, vorzugsweise zwischen 0,1 und 5 Gew.-% Pigmente umfasst.

6. Verfahren nach einem der vorhergehenden Ansprüche, wobei das Polyester-Einsatzmaterial zwischen 0,05 und 1 Gew.-% Farbstoffe, insbesondere zwischen 0,05 und 0,2 Gew.-% Farbstoffe umfasst.

7. Verfahren nach einem der vorhergehenden Ansprüche, wobei das Lösungsmittel in dem Schritt e) des Entfärbens eine Fraktion des aus dem Schritt c) hervorgegangenen Diol-Abstroms, eine Zugabe von verfahrensexternem Diol oder Mischungen davon umfasst.

8. Verfahren nach einem der vorhergehenden Ansprüche, wobei der vorgereinigte Monomer-Abstrom zwischen 30 und 80 Gew.-%, bevorzugt zwischen 50 und 75 Gew.-%, des Gesamtgewichts der Mischung in dem Schritt e) ausmacht.

9. Verfahren nach einem der vorhergehenden Ansprüche, wobei mindestens ein Adsorbens im Schritt e) eine Aktivkohle ist.

10. Verfahren nach einem der vorhergehenden Ansprüche, wobei der Adsorptionsabschnitt in Schritt e) in Gegenwart eines Adsorbens oder von zwei unterschiedlichen Adsorbentien betrieben wird.

11. Verfahren nach einem der vorhergehenden Ansprüche, wobei der Adsorptionsabschnitt zwei oder mehr unterschiedliche Adsorbentien umfasst, wobei die unterschiedlichen Adsorbentien in dem Adsorptionsabschnitt als Gemisch vorliegen oder in Reihe angeordnet sind.

12. Verfahren nach dem vorhergehenden Anspruch, wobei sich jedes der unterschiedlichen Adsorbentien in unterschiedlichen Reaktoren befindet, die in Reihe angeordnet sind.

13. Verfahren nach einem der vorhergehenden Ansprüche, wobei der Adsorptionsabschnitt des Schritts e) im Festbettadsorptionsmodus betrieben wird.

14. Verfahren nach einem der vorhergehenden Ansprüche, wobei der Absorptionsabschnitt des Schritts e) bei einer Temperatur zwischen 100 und 170 °C, bevorzugt zwischen 120 °C und 150 °C betrieben wird.

## Claims

1. Process for depolymerizing a polyester feedstock comprising opaque polyethylene terephthalate (PET), said process comprising:
a) a conditioning step comprising a conditioning section supplied at least with said polyester feedstock, to produce a conditioned stream;
b) a step of depolymerization by glycolysis, which is supplied at least with the conditioned stream and with a supplement of diol in a manner such that the molar amount of diol is adjusted to between 1 to 20 moles of diol per mole of diester in said polyester feedstock, conducted at a temperature of between 180 and 400°C and for a residence time of between 0.1 and 10 h;
c) a diol separation step, which is supplied at least with the effluent from step b), and is conducted at a temperature of between 100 and 250°C, at a pressure less than that of step b), and which produces a diol effluent and a liquid effluent rich in monomers, where said diol separation step is implemented in 1 to 5 successive gas-liquid separation sections, each producing a gaseous effluent and a liquid effluent, where the liquid effluent from the preceding section supplies the subsequent section, the liquid effluent obtained from the last gas-liquid separation section forms the liquid effluent rich in monomers, and all the gaseous effluents are recovered to form the diol effluent;
d) a step of separation of the effluent rich in liquid monomers obtained from step c) into a heavy impurities effluent and a prepurified monomers effluent, which is conducted at a temperature of less than 250°C and a pressure of less than 0.001 MPa with a liquid residence time of less than 10 min, and
(e) a step of decolourizing of the prepurified monomers effluent, comprising at least a section for mixing the prepurified monomers effluent obtained in step d) with a solvent in a manner such that the prepurified monomers effluent represents between 20% and 90% by weight of the total weight of the mixture, and an adsorption section operated at a temperature of between 100 and 200°C and at a pressure of between 0.1 and 1.0 MPa in the presence of at least one adsorbent selected from activated carbons, aluminas and clays, to obtain a purified monomers effluent.

2. Process according to Claim 1, wherein said polyester feedstock comprises at least 50% by weight, preferably at least 70% by weight, more preferably at least 90% by weight of polyethylene terephthalate.

3. Process according to Claim 1 or 2, wherein said polyester feedstock comprises at least 10% by weight, preferably at least 15% by weight, of opaque PET.

4. Process according to any of the preceding claims, wherein said polyester feedstock comprises coloured PET.

5. Process according to any of the preceding claims, wherein said polyester feedstock comprises between 0.1% and 10% by weight of pigments, preferably between 0.1% and 5% by weight of pigments.

6. Process according to any of the preceding claims, wherein said polyester feedstock comprises between 0.05% and 1% of dyes, in particular between 0.05% and 0.2% by weight of dyes.

7. Process according to any of the preceding claims, wherein the solvent in decolourizing step e) comprises a fraction of the diol effluent obtained from step c), a supplement of diol external to the process according to the invention, or mixtures thereof.

8. Process according to any of the preceding claims, wherein the prepurified monomers effluent represents between 30% and 80% by weight, preferably between 50% and 75% by weight, of the total weight of the mixture in step e).

9. Process according to any of the preceding claims, wherein at least one adsorbent in step e) is an activated carbon.

10. Process according to any of the preceding claims, wherein the adsorption section in step e) is operated in the presence of an adsorbent or two different adsorbents.

11. Process according to any of the preceding claims, wherein the adsorption section comprises two or more different adsorbents, said different adsorbents being in a mixture or placed in series in said adsorption section.

12. Process according to the preceding claim, wherein each of said different adsorbents are in different reactors placed in series.

13. Process according to any of the preceding claims, wherein the adsorption section of step e) is implemented in fixed bed adsorption mode.

14. Process according to any of the preceding claims, wherein the adsorption section of step e) is operated at a temperature of between 100 and 170°C, preferably between 120°C and 150°C.
